# EUROPEAN PATENT APPLICATION

(11) **EP 4 129 435 A2**
(43) Date of publication of application: **08.02.2023**
(21) Application number: 22188485.1
(22) Date of filing: 03.08.2022
(51) Int. Cl.: B01D 11/02, A61K 36/185

(54) **METHOD FOR EXTRACTING ACTIVE INGREDIENTS FROM PLANT MATERIAL**

(30) Priority: 03.08.2021 IT 202100020963
(71) Applicant: THC LAB S.r.l., 41037 Mirandola (MO) (IT)
(72) Inventor: LOMBARDI, Annunziata, 41037 Mirandola (MO) (IT)
(74) Representative: Braidotti, Andrea

(57) **Abstract**

Method for the extraction of active ingredients from plant material, and in particular for the extraction of active ingredients from cannabis plants, comprising, in order:
- a step of extraction of at least one active ingredient, preferably THC or CBD in acid forms, by means of a solvent in order to obtain a solution of the active ingredient in the solvent, said extraction step comprising a sonication sub-step,
- a step of removing the solvent from said solution thus obtained to thus obtain the active principle in substantially pure form,
- a step of heat treatment of the active ingredient thus obtained to transform said at least one active ingredient from the acid form to the decarboxylated form,
characterized in that said sonication sub-step involves the use of a probe with a different amplitude ratio based on the type of material starting plant, preferably on the basis of the concentration of the different active ingredients in the starting plant material.

## Description

The present invention relates to a method for extracting active ingredients from plant material, and in particular to a method for extracting active ingredients from cannabis plants.

The Italian legislation considers the possibility of a regulated medical use of Cannabis in the context of chronic pain therapy, to counteract nausea and vomiting caused by chemotherapy and radiotherapy, as an appetite stimulant in cachexia, anorexia, in patients with oncological lack of appetite or suffering from AIDS, in glaucoma and Gilles de la Tourette syndrome, in pathologies involving spasticity associated with pain (multiple sclerosis, spinal cord injury) and in a couple of other pathological conditions.

In the phytocomplex of Cannabis there are over 700 different molecules, of which about a hundred belong to the chemical class of cannabinoids. The molecules of greatest interest from a medical point of view are delta-9-tetrahydrocannabinol ("THC") and cannabidiol ("CBD") in the decarboxylated forms. To date, two isoforms of cannabinoid receptors have been identified in the human body: CB1-type receptors and CB2-type receptors. CB1-type receptors are ubiquitous in the brain, where they exhibit high concentrations especially in certain areas that are related to the many clinical actions of Cannabis. CB2-type receptors are only partially present in the brain, but are more widespread in the periphery, where they demonstrate an action on immune function and inflammation.

THC is a partial agonist of both CB receptors and is responsible for the psychoactive effects of cannabis due to its action on the CB1 receptor; CBD exerts a certain action on CB2-type receptors and other targets such as ion channels and enzymes. Some relevant clinical consequences derive from this knowledge: the action on pain is more powerful when THC and CBD are combined, rather than through the administration of THC alone, and the presence of CBD seems to counteract the psychotropic effects of THC, reducing its adverse effects. It follows that it is very important to know the qualitative-quantitative content of at least the two main cannabinoids mentioned above present in the formulations that are administered to the patient.

The only industrial Cannabis-based medicines that currently have obtained a marketing authorization are Sativex spray and Epidiolex; the first for the treatment of spasticity due to multiple sclerosis, the second for the treatment of rare but severe forms of epilepsy that do not respond to traditional treatments such as Dravet syndrome and Lennox Gastaut syndrome (LGS).

Sativex is a oromucosal spray that delivers 2.7 mg of THC and 2.5 mg of CBD per serving, with a maximum daily dose of 32.4 mg of THC and 30 mg of CBD. Epidiolex is an oral solution that contains 100 mg of Cannabidiol in refined sesame oil.

The legislation in force in Italy provides that the administration of Cannabis for medical use can take place orally or by inhalation. As regards the galenic preparations, relative to the oral administration route, the pharmaceutical form to be considered as the first choice is the decoction, but administration in the form of an extract is also envisaged. The inhalation route of administration is to be considered if oral administration does not produce the desired pharmacological effects or when the treating physician deems it appropriate. With reference to the decoction it has been observed that the acidic cannabinoids are more present than the corresponding decarboxylated cannabinoids, consequently with the decoction the patients do not take a quantity of THC and CBD useful for therapeutic purposes since usually a maximum of 500 ml of decoction/day are taken. A quantitative analysis of the content of THC and CBD for decoctions prepared according to the indications provided by the Ministry of Health (100 mg of Cannabis per 100 ml of water) has in fact provided the data shown in table 1 and allowed to calculate a ratio between the acid fraction and the decarboxylated fraction on average equal to 2.30 ± 0.218 for THC and 4.85 ± 0.315 for CBD.

**Table 1: Content of active ingredients in a decoction made according to the recipes provided by the Ministry of Health**

| Method | Active molecule | Number of analyzed samples | Average of the content of active molecules mg/100 ml | Standard deviation | Content of active molecules: minimum value mg/100ml | Content of active molecules: maximum value mg/100ml |
|---|---|---|---|---|---|---|
| A1 (100mg of Cannabis in 100ml of water) | THC | 39 | 0,87 | 0,41 | 0,26 | 2,47 |
| | CBD | | 1,07 | 0,43 | 0,27 | 2,42 |
| | THCA | | 1, 81 | 0.55 | 0.88 | 3.09 |
| | CBDA | | 4.83 | 1.07 | 2.59 | 7.71 |
| | CBN¹ | | 0.02 | 0.03 | 0.00 | 0.15 |
| A2 (200mg of Cannabis in 200ml of water) | THC | 6 | 0.72 | 0.15 | 0.53 | 0.88 |
| | CBD | | 1.01 | 0.14 | 0.86 | 1.24 |
| | THCA | | 1.81 | 0.89 | 0.58 | 2.52 |
| | CBDA | | 5.18 | 0.91 | 3, 87 | 6.38 |
| | CBN | | ND | ND | ND | ND |
| A3 (300mg of Cannabis in 300ml of water) | THC | 6 | 0,83 | 0,26 | 0,59 | 1,31 |
| | CBD | | 1,09 | 0,26 | 0,90 | 1,62 |
| | THCA | | 1 , 91 | 0.78 | 0.92 | 2.60 |
| | CBDA | | 5.36 | 0.63 | 4.29 | 6.12 |
| | CBN | | ND | ND | ND | ND |

| | | | | | | |
|---|---|---|---|---|---|---|
| ¹ CBN: cannabinolo | | | | | | |

As for the oily preparations for oral use, the methods currently known and used for the preparation of galenic preparations based on Cannabis are essentially five: the one called "RomanoHazekamp", the one called "Citti-Cannazza", the one called "SIFAP" , the one called "Calvi at all." and the one called "Pacifici".

The first two methods require that the cannabis is previously chopped and then mixed with olive oil. The mixture obtained is then heated (for two hours in a boiling water bath for the "Romano-Hazekamp" method, for two hours at 110°C for the "Citti-Cannazza" method) and then filtered to obtain the oily extract. The "SIFAP" method, on the other hand, requires the cannabis to be chopped and then preheated to 115°C for 40 minutes. For preheating the drug is distributed in a layer with a height of 1 cm. The cannabis is then transferred to the olive oil and further crushed with a turbo emulsifier for 3 minutes. The mixture of cannabis and olive oil is then heated in a boiling water bath for 40 minutes, filtered and added with 0.02% butylhydroxyituoluene (BHT). All the methods in question provide that the weight (g)/volume (ml) ratio between drug and solvent is equal to 0.1:1. The "Pacifici" method involves preheating the plant material at 145°C for 30 minutes and subsequent heating in a boiling water bath for two hours. The "Calvi" method does not require the preheating of the plant material but the use of ultrasounds.

On the basis of what is reported in the literature, if we analyze the quantities of active molecules that can be obtained by applying the first three methods mentioned above, the following results are obtained for the FM2 variety of Cannabis (table 2):

**Table 2: Comparison of yields obtained with different known extraction methods**

| METHOD | % THC | % CBD | % THCA | % CBDA |
|---|---|---|---|---|
| Romano-Hazekamp | 0.17 ± 0.09 | 0.05 ± 0.03 | 0.18 ± 0.06 | 0.47 ± 0.10 |
| Citti-Cannazza | 0.24 ± 0.08 | 0.23 ± 0.11 | 0.24 ± 0.14 | 0.67 ± 0.25 |
| SIFAP | 0.37 ± 0.08 | 0.70 ± 0.19 | 0.07 ± 0.07 | 0.15 ± 0.09 |

The most effective method, the one called "SIFAP", allows at most to obtain an oil whose title in THC is equal to 0.37% ± 0.08 (3.38 mg/ml) and the title in CBD is equal to 0.70% ± 0.19 (6.40 mg/ml).

The known processes for the extraction of THC and CBD from cannabis in the form of oil do not therefore allow a significant extraction of the aforementioned active ingredients.

US 2019/038663 and US 2020/398184 describe two methods for the extraction of THC, CBD and other compounds from a cannabis-based raw material, both of which involve a sonication step of the starting material.

However, these two solutions are not fully satisfactory as they each provide for the use of the same sonication instrument , therefore not obtaining the advantages described in the present patent application and originating from the selection of an appropriate sonication instrument on the basis of the nature of the starting material.

The object of the invention is to propose a method for the extraction of active principles from plant material, and in particular from cannabis plants, which allows to overcome the drawbacks of the known solutions.

Another object of the invention is to propose a method which is simple and quick to implement.

Another object of the invention is to propose a method that allows the active principles to be extracted efficiently from the plant material.

Another object of the invention is to propose a method which is easily reproducible.

Another object of the invention is to propose a method which is safe for a user.

Another object of the invention is to propose a method which allows to reduce as much as possible the extraction of unwanted compounds.

Another object of the invention is to propose a method which is selective.

Another object of the invention is to propose a method which allows to vary with precision the ratio between the acid form and the decarboxylated form of the active principles.

Another object of the invention is to propose a method which allows to reduce the use of reagents.

Another object of the invention is to propose a method which allows to obtain a final solution with the required title.

Another object of the invention is to propose a method which allows to extract all the components required by the plant material.

Another object of the invention is to propose a method which limits the dispersion of volatile components.

Another object of the invention is to propose a method which allows to use different solvents in the final preparation.

Another object of the invention is to propose a method which allows to obtain different concentrations of the active ingredients.

Another object of the invention is to propose a method which allows to prepare small and large quantities of plant material, reducing processing losses to a minimum.

Another object of the invention is to propose a method which allows to obtain stable preparations in the time of use.

Another object of the invention is to propose a method which allows to optimize the processing costs, the time taken to finalize the process and the energy required.

Another object of the invention is to propose a method which allows to obtain a homogeneous solution.

Another object of the invention is to propose a method which does not use toxic or harmful solvents.

All these purposes, whether considered alone or in any combination thereof, and others that will result from the following description, are achieved according to the invention with a method for the extraction of active principles from plant material, and in particular from cannabis plants. according to claim 1, and with the use of a product according to claim 15.

The present invention is further clarified hereinafter in some of its preferred embodiments, given purely by way of non-limiting example.

The invention relates to a method for extracting active ingredients from plant material, and in particular from cannabis plants.

In particular, the method according to the invention allows to extract the active ingredients from the plant material, in particular the active ingredients such as acid THC and acid CBD in the case of the cannabis plant, and to chemically transform them in order to obtain relevant molecules from a medical point of view, such as THC and CBD in the case of cannabis.

The starting product is represented by plant material, which for example can be composed of preferably female inflorescences of the cannabis plant, which can be of the indica or sativa or hybrid variety. It is clear that the starting plant material may also include other parts of the cannabis plant, such as leaves and/or stems, or any portion of the plant that may contain the required active ingredients, and in particular acid THC and/or acid CBD.

Appropriately below we will talk about varieties with a higher THC content as well as varieties in which the THC content is much higher than that of CBD, as defined by the pharmacopoeia. In particular, with varieties with a higher THC content, we mean varieties that include THC in a percentage higher than 12%, and CBD lower than 1%. Among these we recognize the varieties with a high THC content, which include THC in a percentage greater than 16%, and those with a medium THC content, which include THC in a percentage between 12 and 16%.

In particular, the varieties with a higher THC content include for example the following varieties: Bedrocan, Bedica, Pedanios 22/1, FM1, Bedrobinol.

Appropriately, below we will talk about varieties with a higher CBD content as a variety in which the CBD content is much higher than that of THC, as defined by the pharmacopoeia. In particular, with varieties with a higher CBD content we mean varieties that include CBD in a percentage higher than 7%, and THC lower than 1%.

In particular, the varieties with a higher CBD content include for example the following varieties: Bedrolite, Aurora 1/12.

Appropriately below we will talk about varieties containing both THC and CBD as well as varieties in which the content of THC and CBD is comparable and/or similar, as defined by the pharmacopoeia. In particular, with varieties containing both THC and CBD we mean varieties that include THC and CBD in a percentage THC 5-8%, CBD 6-12%

In particular, the varieties containing both THC and CBD include for example the following varieties: FM2, Bediol.

Advantageously, the process according to the invention can provide for a preliminary storage step of the starting plant material. In particular, the preliminary step can be carried out by keeping the starting plant material in a refrigerated place, and preferably in the freezer at a temperature of about -20°C. In this way it is possible to preserve it for a long time without the active ingredients contained in it being subjected to reactions, and in particular to decarboxylation reactions at room temperature, thus modifying the properties of the starting product, and consequently those of the final product.

Subsequently, the plant material can be subjected to a grinding step configured to reduce the size of the starting product and improve the efficiency of the extraction process. Preferably, the grinding step can be carried out in a traditional grinding device, which for example can be a ball mill or in a pin mill. Preferably, the grinding device can be made at least partially of metal, in order to prevent the formation of electrostatic charges in the ground plant material, so as to simplify its removal from the grinding device itself.

Preferably the grinding chamber used is previously stored at -20°C for 3 hours before use, just to reduce the loss of plant material during the grinding process, alternatively the process is carried out with the use of dry ice in the grinding chamber.

Conveniently, the grinding parameters can be those shown in Table 3, and preferably they can be modified according to the conditions of the starting plant material.

**Table 3: Grinding parameters for different types of starting products Starting**

| plant material | Rotation speed (rpm) | Time (s) | Impulses |
|---|---|---|---|
| Whole inflorescences | 10.000 | 20 | 2 |
| Granulated inflorescences | 10.000 | 10 | 2 |
| Pre-chopped inflorescences | N/D | N/D | N/D |

Advantageously, the times of the grinding step and/or the parameters of the grinding device allow to obtain an optimal compromise between the granulometry of the product obtained and the protection of any volatile products contained in the inflorescences, such as for example terpenes and/or other active ingredients.

Preferably, the grinding step can be carried out while the plant material is still at a low temperature to minimize the loss of volatile compounds and/or active ingredients.

Clearly the parameters can be modified according to the conditions of the starting plant material and the size of the inflorescences.

Conveniently, if the starting product includes whole inflorescences, a further grinding step can be implemented. Advantageously, said further grinding step can be carried out by immersion with a turbo-emulsifier, in order to homogenize the starting vegetable material.

Advantageously, the granulometry of the ground plant material can be controlled by means of a suitable fine-mesh sieve.

In one embodiment, the grinding step may comprise a step of treating the plant material with dry ice in a ratio of 1:10. In particular, the plant material is placed in a container together with dry ice and is repeatedly forced into a hopper in order to separate the most homogeneous part of the inflorescences, the trichomes, and richer in active ingredients and functional substances from the residual part of the processing, stems and leaves. In this way, therefore, it is possible to improve the efficiency of the extraction process and to refine the starting plant material, making the final product purer and more pleasant to the taste.

Subsequently, the ground plant material can be subjected to a solvent extraction step of the required active ingredients. In this step the ground plant material can be immersed in a suitable solvent configured to solvate the required active ingredients which are present in the ground plant material.

In a preferred embodiment, the solvent may be ethanol, which is safe to use, and non-toxic, as well as being commonly used as a solvent in medicinal and galenic preparations.

In a preferred embodiment the ethanol can be used in concentrated form, ie with a purity higher than 90%, and preferably higher than 96%, and more preferably higher than 99%.

Preferably the solvent can be used cold, for example at a temperature of -20°C and more preferably at a temperature of -40°C.

Advantageously, the ratio between the weight in grams of ground vegetable material and the volume in millilitres of solvent can be less than or equal to 1:30, for example 5g of ground material in 150 ml of ethanol. In this way it is possible to obtain optimal yields with a lower consumption of solvent than for example what is reported in the study by Luigi L. Romano and Arno Hazekamp method 3 in which a 1:40 ratio is used.

In particular, if 96% ethanol is used, the ratio between the weight in grams of ground vegetable material and the volume in millilitres of solvent can be about 1:30.

Conveniently, the weight in grams of chopped plant material and the volume in millilitres of solvent can be about 1:20 for quantities up to about 25g, 1:15 for quantities between about 25 and about 50g, and about 1:10 for quantities between about 50 and about 100g.

Conveniently, the extraction step can be carried out by maintaining the solvent and the minced vegetable material at a low temperature, for example at -20°C and preferably -40°C. In this way it is possible to reduce the loss and/or degradation of volatile material. Furthermore, at temperatures below 0°C any water contained in the solvent and/or in the crushed plant material freezes. In this way the water can no longer play its role as a solvent and this helps to limit the extraction of chlorophyll (a polar molecule well soluble in water) from the chopped plant material, which would give the final product a bitter and therefore undesirable taste. Further, the use of low temperatures reduces the evaporation of the solvent and therefore reduces the risk of fire during the process.

Advantageously, the extraction step can be carried out in a suitable container, preferably made of thermally insulating material in order to limit the heating of the solvent and/or of the plant material. In particular, it can be made of glass. Conveniently it can have a conical shape with a narrow entrance, in order to minimize the exchange of air between the inside and the outside, as well as the evaporation of the most volatile component of the plant. Furthermore, the container can be immersed in a bath of cold liquid, or refrigerated by other means.

Preferably, during the extraction step, the temperature of the solvent and/or of the chopped plant material can be kept below 30°C, and preferably below 15°C.

In a preferred embodiment the inlet of the container can be in an isolated and/or sealed fluidic connection with a condenser, configured to allow the condensation of any vapors that could be released during the extraction step. Advantageously, these vapors, once recondensed, can be reintroduced into the container. Conveniently, therefore, the environment in which the extraction step takes place can be sealed from the outside, and this allows the characteristics of the material to be kept as unaltered as possible.

For example, the container can be represented by a conical flask, to whose inlet one end of a bubble coolant can be connected. A refrigerated fluid is passed through the interstice of the bubble refrigerant, which allows the vapors coming out of the flask to condense and make them fall back into the flask itself. Conveniently, the end of the refrigerant that is not inserted in the flask can be sealed to prevent vapors from escaping.

In a preferred embodiment, the extraction step can be carried out comprising a sonication sub-step - ie with a process called "sonication". In particular, for example, the container can have the inlet for a probe configured to vibrate at high frequency, and which can be immersed, at least partially, in the solvent and/or in the plant material. In this way the probe can vibrate the solvent and make the extraction step faster and more effective.

Conveniently, sonication can be performed at the maximum potential of the probe in order to minimize the duration of the extraction step. For example, the probe can emit a power of 400W, at a frequency of 24KHz.

Conveniently, sonication can be accomplished by using an immersion probe.

There are different types of probes and their choice is generally influenced by the sample volume, as shown in the table.

| PROBE TYPE | DIAMETER | SAMPLE VOLUME | AMPLITUDE RATIO |
|---|---|---|---|
| AMPLITUDE S 24D7 | 7mm (38.5mm²) | 20ml - 500ml | 1: 4 |
| S24D14D | 14mm (213mm²) | 50ml - 1000ml | 1: 2.5 |
| S24D22L2D | 22mm (400mm²) | 100ml - 4000ml | 1: 0.9 |

As known, with amplitude or amplitude A, we mean the distance (preferably longitudinal) by which the probe moves during the sonication process in order to generate the sound wave. In particular, it can be measured in microns (µm). With "amplitude ratio" we mean the ratio between the amplitude of the displacement generated by the sonicator and that actually obtained at the tip of the probe used. In particular, therefore, for example, the S 24D7 probe can emit, at its tip, an amplitude equal to approximately 4 times that emitted by the sonicator.

However, it has been noted and verified that the choice of the probe influences the extraction efficiency; in particular, according to the type of cannabis and the type of probe, the extraction parameters change.

| CANNABIS | PROBE | EXTRACTION TIME |
|---|---|---|
| HIGH THC | S24d7 | 7-10 minutes |
| MEDIUM THC | S24d14D | 10-13 minutes |
| THC: CBD | S24d22L2D | 13-15 minutes |
| HIGH CBD | S24d22L2D | 15-20 minutes |

Advantageously, suitable stirring means may also be present, such as for example a magnetic stir bar subjected to a magnetic field which sets it in rotation, in order to homogenize the solvent and the vegetable material or a paddle stirrer.

Conveniently, the extraction step can last for a predefined time interval depending on the granulometry and/or the quantity of ground plant material and/or on the basis of the quantity of the different active ingredients present in the starting plant material. In particular in the case of cannabis plants with a high or medium THC content - ie THC ≥ 10% - it can last up to about 13 minutes, in the case of cannabis plants with a high CBD content - ie CBD ≥ 6% - it can last up to about 20 minutes, and for cannabis plants with comparable THC and CBD content it can last an intermediate time, for example about 15 minutes. In the case of varieties such as the so-called FM1 and FM2 it can last less than 10 minutes, and in particular about 7 minutes.

Subsequently, the method according to the invention can provide a filtration step aimed at separating the solid residues of the minced vegetable material from the solvent containing the active ingredients. In particular, the filtration step can be carried out by passing the solvent with the ground plant material in suspension through a filter configured to retain the ground plant material. Advantageously, said filtration step can be carried out with the help of a vacuum pump in order to make it faster. Conveniently, in particular in the case of large quantities of material, a second extraction step can be carried out with a pressure press.

Furthermore, the method according to the invention can provide for a further extraction step, aimed at extracting one or more active ingredients from the ground plant material which are preferably different from those which are extracted in said extraction step. Conveniently, said further extraction step can be carried out before or after said extraction step and said filtration step. In particular, for example, the plant material which has been obtained after said filtration step can be subjected to said further extraction step.

For example, said further extraction step can provide for immersing the chopped plant material in a further solvent which can preferably be different from the solvent used in the extraction step. Preferably, the further solvent can be configured to selectively extract one or more active principles present in the starting plant material which are not extracted from the solvent. For example, the further solvent can be a lipophilic solvent, such as for example olive oil, MCT oil, or a mixture thereof, or an emulsion, or in any case a lipophilic solvent suitable for human consumption. Advantageously, in this way it is possible to extract the non-polar active ingredients which it was not possible to extract with ethanol.

Conveniently, said further extraction step can comprise a sonication sub-step, substantially similar to that carried out for said extraction step.

Subsequently, the method according to the invention can provide a further filtration step substantially similar to the filtration step, configured to separate the minced plant material from the further solvent. Preferably, said further filtration step can be carried out by using a press filter.

Conveniently, the extraction, further extraction, filtration and further filtration steps can be repeated an appropriate number of times, and for example each can be repeated up to three times. Order: extraction, filtration, further extraction, filtration and finally dilution.

Subsequently, the method according to the invention can provide for a step of removing the solvent from the solution obtained after the filtration step and/or further filtration configured to remove the solvent and/or the further solvent, and thus obtain the active ingredients extracted in the form substantially pure.

Subsequently, the method according to the invention provides for a treatment step of the extracted active ingredients. In particular, the treatment step can be a heat treatment step aimed at modifying the chemical nature of the extracted active ingredients. In particular, it can include a thermally activated decarboxylation process, which can allow the acid forms of the active ingredients extracted from the plant material - in particular acid THC and acid CBD - to transform into alcoholic forms - in particular THC and CBD.

Conveniently, the decarboxylation process can be carried out by inserting the active ingredients inside a heated environment, such as an oven. Preferably the oven can be static, or alternatively it can be a vacuum oven, in order to reduce the loss of volatile components such as terpenes. Preferably, the oven may not be ventilated, in order to reduce the loss of volatile components.

Conveniently, the process parameters, i.e. temperature and time, can be varied according to the content of active ingredients, and in particular to the different proportion of the different active ingredients, which can influence the kinetics of decarboxylation, and/or its particle size, and/or the percentage of active ingredients in acid form that you want to transform into alcoholic form, and/or the amount of plant material.

Advantageously, the temperature can be between 100 and 150°C, and preferably it can be kept around about 130°C. Advantageously, the chemical treatment step can last from 30 to 80 minutes, and in particular the parameters can be those indicated in table 4.

**Table 4: Decarboxylation parameters**

| CATEGORIES VARIETIES | TEMPERATURE (° C) | TIME (minutes) Total decarboxylation | TIME (minutes) Partial decarboxylation |
|---|---|---|---|
| THC varieties (THC> 10%) | 130 | 30 - 65 | 15-30 |
| THC and CBD varieties | 130 | 40 - 70 | 20 - 40 |
| CBD varieties | 130 | 45 - 80 | 30 - 50 |

Furthermore, the method according to the invention can provide for a dispersion step in a suitable carrier or carrier, aimed at dispersing the active principles extracted during the extraction step in a suitable carrier. Conveniently said dispersion step in a suitable carrier can be carried out after said extraction step.

For example, said dispersion step in a suitable carrier can provide for immersing the active principles obtained from the extraction from the starting plant material in a carrier which can preferably be different from the solvent used in the extraction step. For example, the carrier can be a lipophilic solvent, such as for example olive oil, MCT oil, or in any case a lipophilic solvent suitable for human consumption.

Conveniently, the dispersion step in a suitable carrier can provide a sonication sub-step. Advantageously, said sonication sub-step can provide for a sonication of the carrier before the active ingredients extracted in said extraction step are added to it. In this way it is possible to avoid heating the active ingredients, avoiding alterations or losses of the same. Alternatively, said sonication sub-step can be carried out after the active ingredients have been added to said carrier, in order to improve their dispersion.

Conveniently, moreover, the dispersion step in a suitable carrier can comprise a sub-step of reduction of the size of the drops and/or particles of the active ingredient, and preferably a micronization step, which allows to reduce the size of the drops and/or particles of active ingredient below a predefined and/or user-selected threshold.

Subsequently, the method according to the invention can provide a further filtration step substantially similar to the filtration step, configured to separate the minced plant material from the further solvent. Preferably, said further filtration step can be carried out by using a press filter.

Conveniently, the extraction, filtration, further filtration and dispersion steps in a suitable carrier can be repeated an appropriate number of times. Preferably the dispersion step in a suitable carrier can always follow the extraction and/or further extraction and/or filtration step.

Subsequently, the method according to the invention can provide for a step of removing the solvent from the solution obtained after the filtration step and/or further filtration configured to remove the solvent and/or the further solvent, and thus obtain the active ingredients extracted in the form substantially pure.

For example, said solvent removal step can provide for the evaporation of the solvent, preferably carried out by means of a rotary evaporator. Advantageously, the evaporation of the solvent can take place slowly and gradually, and at a low temperature in order to prevent any degradation of the active ingredients dissolved therein. Advantageously, for this purpose, evaporation can take place at a pressure lower than the ambient one, for example at a pressure between 30 and 170mbar and at a temperature of 40°C if the solvent is ethanol.

Advantageously, the pressure can be gradually lowered during evaporation.

Preferably, the solvent can be recondensed in a suitable container in order to be reused in a subsequent extraction step.

Subsequently, a dilution step of the active ingredients treated in the chemical treatment step can be carried out with a suitable diluent. Advantageously, the diluent can be configured not to alter the physico-chemical properties of the active ingredients, and be compatible with internal use in humans. For example, said eluent can be an oil, such as for example olive oil or MCT oil. In the embodiment which provides for the dispersion step in a suitable carrier, the carrier used in that step can be used as an eluent, in this way it is possible to efficiently recover the compounds extracted in the dispersion step in a suitable carrier. Advantageously, the quantity of eluent used can be measured in order to obtain a solution containing the appropriate concentration of active principle.

Conveniently, said dilution step can be carried out by keeping the diluent under agitation, in order to make the solution homogeneous.

Advantageously, said dilution step can be divided into two sub-steps, a first sub-step in which most of the diluent is added, for example 90% of the diluent and a second sub-step in which the remaining part of the diluent is added.

Conveniently, between the two dilution sub-steps, a chemical analysis step of the solution obtained can be provided, and in particular a titration step of the solution obtained, in order to verify its concentration. Advantageously, subsequently, the remaining part of the diluent which is added during the second dilution sub-step can be accurately measured in order to obtain the desired concentration.

Advantageously, the residual solvent of the second extraction carried out on the plant material can be used for dilution.

Advantageously, the final preparation can be further micronized with a sonication instrument in order to increase its bioavailability.

Advantageously, it is possible to add excipients which improve its absorption.

Advantageously, said extract can be emulsified in order to increase its bioavailability.

From what has been said it is clear that the method according to the invention is advantageous, and indeed optimal, since:
- allows to selectively extract the active ingredients required by minimizing the amount of compounds that are lost during the process,
- allows to minimize the amount of reagents used,
- allows you to accurately check the chemical composition of a drug produced,
- allows to obtain a high extraction efficiency,
- allows to recover the extraction solvent,
- allows to obtain different concentrations starting from the same plant matrix,
- allows the choice of multiple solvents as a vehicle,
- saves energy, solvents, time,
- allows to carry out the extraction at room temperature.

## Claims

1. Method for the extraction of active ingredients from plant material, and in particular for the extraction of active ingredients from cannabis plants, comprising, in order:
- a step of extraction of at least one active ingredient, preferably THC or CBD in acid forms, by means of a solvent in order to obtain a solution of the active ingredient in the solvent, said extraction step comprising a sonication sub-step,
- a step of removing the solvent from said solution thus obtained to thus obtain the active principle in substantially pure form,
- a step of heat treatment of the active ingredient thus obtained to transform said at least one active ingredient from the acid form to the decarboxylated form,
**characterized in that** said sonication sub-step involves the use of a probe with a different amplitude ratio based on the type of material starting plant, preferably on the basis of the concentration of the different active ingredients in the starting plant material.

2. Method according to claim 1 **characterized in that** said sonication sub-step lasts less than 20 minutes, and preferably less than 15 minutes.

3. Method according to one or more of the preceding claims **characterized in that** said sonication sub-step lasts:
- a time of less than about 13 minutes for plant material with a higher THC content,
- a time of less than about 15 minutes for plant material with similar THC and CBD content, and
- a time of less than about 20 minutes for plant material with a higher CBD content.

4. Method according to one or more of the preceding claims **characterized in that** said sonication sub-step involves the use of a probe with an amplitude ratio of:
- 1: 4 for high THC plant material,
- 1: 2.5 for plant material with medium THC content,
- 1: 0.9 for plant material with comparable THC and CBD content and plant material with higher CBD content.

5. Method according to one or more of the preceding claims **characterized in that** the choice of the amplitude ratio of said probe does not depend on the quantity of solvent and/or quantity of plant material included in said extraction step of at least one active principle.

6. Method according to one or more of the preceding claims **characterized in that** said step of extraction of at least one active principle is carried out at a temperature lower than 30°C, preferably lower than 15°C.

7. Method according to one or more of the preceding claims **characterized in that** said solvent is ethanol, and preferably ethanol with a purity equal to or greater than 96% and maintained at a temperature below -20°C, and preferably at a temperature below -40°C.

8. Method according to one or more of the preceding claims **characterized in that** said step of extraction of at least one active principle takes place in a sealed container.

9. Method according to one or more of the preceding claims **characterized in that** said solvent is ethanol, and preferably ethanol with a purity higher than 96%, and more preferably equal to 99% and which is maintained at a temperature lower than - 20°C, and preferably below -40°C, and that the ratio between ml of solvent and g of weight of the plant material is less than or equal to 30: 1.

10. Method according to one or more of the preceding claims **characterized in that** it comprises, preferably between said step of extracting at least one active ingredient with a solvent and said step of removing the solvent, a step of further extraction of at least one active ingredient with a further solvent, and that said further solvent is olive oil, MCT oil, or a mixture thereof, or an emulsion, and that said solvent removal step is configured to remove also said further solvent.

11. Method according to one or more of the preceding claims **characterized in that** it comprises a dispersion step of the active principle thus treated in a carrier which is different from said solvent.

12. Method according to one or more of the preceding claims **characterized in that** said dispersion step in said carrier comprises a sonication sub-step of said carrier and said sonication sub-step preferably takes place before or after the addition of said at least one active principle to said carrier.

13. Method according to one or more of the preceding claims **characterized in that** said dispersion step in said carrier comprises a sub-step for reducing the size of the drops and/or particles of said active ingredient, and preferably a micronization step, to thus reduce the size drops and/or particles of active ingredient below a predefined and/or user-selected threshold.

14. Method according to one or more of the preceding claims **characterized in that** the ratio between the weight in grams of ground vegetable material and the volume in milliliters of solvent can be less than or equal to 1:15, and preferably less than or equal to 1:30.

15. Use of a product made by a method according to claims 1-14 in the treatment of pain, to counteract nausea and vomiting caused by chemotherapy and radiotherapy, as an appetite stimulant in cachexia, anorexia, in oncological or AIDS inappetent patients glaucoma and Gilles de la Tourette syndrome, in diseases involving pain associated spasticity (multiple sclerosis, spinal cord injury), and in a couple of other pathological conditions.
